# EUROPEAN PATENT SPECIFICATION

(11) **EP 2 222 310 B1**
(45) Date of publication and mention of the grant of the patent: **03.08.2016**
(21) Application number: 08806589.1
(22) Date of filing: 13.10.2008
(51) Int. Cl.: A61K 31/702, A61K 35/74, A61K 45/06, A61K 47/46, A61P 31/04, A61K 36/45, A23L 33/10, A23L 19/00, A23L 2/52

(54) **Preparation for treating intestinal infection comprising oligosaccharides and insoluble cellular material**
Präparation zur Behandlung von intestinaler Infektion, enthaltend Oligosaccharid und unlösliches Zellmaterial
Préparation utile pour traiter une infection intestinale, ladite préparation comprenant des oligosaccharides et une matière cellulaire insoluble

(30) Priority: 11.10.2007 GB 0719882; 02.06.2008 GB 0810006; 08.09.2008 GB 0816361
(43) Date of publication of application: 01.09.2010
(73) Proprietor: Promovita ingredients limited, Esher Surrey KT10 9PN (GB)
(72) Inventor: PRIEST, Leslie, Crewe, Cheshire CW1 5UF (GB); FOWLER, Vernon, Crewe, Cheshire CW1 5UF (GB); HILLMAN, Kevin, Crewe, Cheshire CW1 5UF (GB)
(74) Representative: Jones, Bruce Graeme Roland
(86) International application number: PCT/GB2008/003459
(87) International publication number: WO 2009/047537

(56) References cited:
- EP-A- 1 795 204
- WO-A-01/65949
- WO-A-03/106472
- WO-A-2005/053425
- WO-A-2005/060937
- WO-A-2005/092127
- WO-A-2006/039768
- WO-A-2007/096838
- US-A1- 2004 126 440
- OFEK I ET AL: "Anti-adhesion therapy of bacterial diseases: Prospects and problems" FEMS IMMUNOLOGY AND MEDICAL MICROBIOLOGY, ELSEVIER SCIENCE B.V., AMSTERDAM, NL, vol. 38, no. 3, 15 January 2003 (2003-01-15), pages 181-191, XP009091590 ISSN: 0928-8244

## Description

### BACKGROUND TO THE INVENTION

### 1 FIELD OF THE INVENTION

The present invention relates to the treatment of intestinal pathogens. Three of the best known such pathogens which are harmful to humans include *Salmonella, Clostridium difficile* ("*C*. *difficile*") and certain strains of *Escherichia coli* ("*E. coli*"). *C. difficile* infection is increasing currently within hospitals, resulting in a dramatic increase in cases of patients with serious and, in some instances, life-threatening symptoms. Present treatment protocols involve treatment based primarily on the use of antibiotics, such as metronidazole, vancomycin and linezolid. *C. difficile,* however, is resistant to most antibiotics. In addition it is a commensal bacterium (i.e. a bacterium which lives within the intestine of its host without harming its host) in a section of the population. Patients who are asymptomatic carriers of *C. difficile* may, therefore, if treated with "wide-spectrum" antibiotics in conjunction with another illness, say, suddenly develop very serious symptoms of *C. difficile* infection, such as pseudomembranous colitis. This occurs because the wide-spectrum antibiotics reduce the levels of normal intestinal flora while having no effect on the *C. difficile,* meaning that the *C. difficile,* with reduced competition, flourish.

*Salmonella* infection is most frequently regarded as a food-borne illness. Treatment of *Salmonella* has been by antibiotic. Such treatment can, however, give rise to the problems referenced above in relation to *C. difficile* infection, while the long-term usage of antibiotics in both the poultry and beef industries may have created a strain of *Salmonella* which is potentially resistant to antibiotics.

Routes of infection by pathogenic strains of *E. coli* are varied. This organism is usually spread by faecal contamination and can be passed on through food, water, or from the environment. A treatment which suppresses the activity of the coliform group of bacteria within the intestine would increase the host's resistance to infection by these pathogens.

### 2. DESCRIPTION OF RELATED ART

It is known that the human intestine and, in particular, the colon contains many microflora which are thought to have a symbiotic relationship with their human host by aiding digestion and preventing growth of harmful species. A particular group of such helpful organisms, thought to have a beneficial effect in aiding a human host fight many forms of infection, is the lactic acid bacteria group, hereafter referred to by their primary members, *Lactobacillus* spp.. *Lactobacillus* is thought to help fight infectious bacteria, such as *C. difficile,* in a number of ways. Firstly, *lactobacillus,* when acting on certain fermentable substrates, causes a lowering of the pH of the gut contents which inhibits the growth of the pathogen. By contrast, *C. difficile* flourishes in an environment with a more neutral pH, meaning that the presence of *lactobacillus* will result in an environment which is deleterious to *C. difficile.* Secondly, *lactobacillus* may have an antibacterial effect which acts against competing bacteria such as *C. difficile.* An increase in *lactobacillus* within a host intestine which is infected with *C. difficile* can, therefore, result in a diminution of symptoms associated with *C*. *difficile* infection. A superficially attractive solution to the problem of fighting intestinal infection, therefore, is to introduce additional *Lactobacilli.* However, it has been found that this is rarely effective for the reason that such additional quantities of bacteria are not sustainable, and that the added species might not be adapted for survival in that host's intestine. A host's population of *Lactobacillus* is primarily dependent upon the conditions which are suitable for it to flourish. The treatments described here act to enhance the host's own population of *Lactobacillus* spp., which are already adapted for survival in the intestine and will therefore rapidly increase in numbers and activity if provided with suitable nutrients.

*Lectobacilli* are known to flourish in environments which are rich in specific sugars. Simple sugars, however, are difficult to transport to the latter parts of an intestine because they are very largely absorbed and consumed very early in the digestive process. Thus, by the time a food bolus has reached the colon - the location where the target infection is largely located - it is unlikely to contain sugars in any large quantities Feeding sugar to a host will not, therefore, provide the requisite conditions for *Lactobacillus* in the lower intestine. Certain oligosaccharides, which are polymers containing typically between three and ten simple sugars, however, are more difficult for the early digestion to break down. The administration of particular oligosaccharides is reported to provide an increase in the number of 'friendly' bacteria and simultaneous reduction in the population of harmful bacteria.

### SUMMARY OF THE INVENTION

One aspect of the present invention provides an appropriate environment for the culture of *lactobacillus* in the lower intestine by the use of oligosaccharides. According to a first aspect of the present invention there is provided the use of a galactooligosaccharide and pulp of hemi-cellular material from cranberry in the creation of a preparation for use in treatment of one of clostridium difficile and salmonella.

The preparation may be created as a relatively long-life food preparation which enables easy transportation and consumption, such as packaged, dried fruit or fruit bars (whether contained within an air-tight wrapping or otherwise), for example. Alternatively, the preparation may be fresh and in the form of a drink (such as, for example, the kinds of drink which are currently in vogue under the epithet 'smoothie') or a yoghurt drink. The preparation may, in one embodiment, be used in the treatment (whether by prophylaxis or remedial infection) of intestinal infection by *C. difficile* or *Salmonella.* When used as a prophylactic, it enhances the ability of the intestinal microflora to repel subsequent infections.

Where the preparation includes a pulp, according to one embodiment, the pulp is made of whole, milled cranberries. Fruit pulp, being largely composed of chemically complex structural components of plant cells which are therefore more difficult for the early digestion to break down, assists in the delivery of larger amounts of oligosaccharide to the lower intestine.

A preferred aspect of the present invention provides the use of hemi-cellular material from cranberries in the creation of a preparation for the treatment for enterotoxin released in the human alimentary canal by *clostridium difficile.*

The present invention further preferably additionally provides a preparation for the treatment of harmful intestinal bacteria comprising one or more of *lactobacillius salivarius, lactobaciulls brevis, lactobacillus buchner* each of which have been found to flourish beneficially in the presence of galacto-oligosaccharide. In a preferred embodiment, the preparation is for the treatment of *salmonella.*

### BRIEF DESCRIPTION OF DRAWINGS

Embodiments of the present invention will now be described, by way of example, and with reference to the accompanying drawings in which:
Fig. 1 is a schematic representation of a pig-gut fermentation vessel used to test the effects of various substrates for the delivery of oligosaccharides; and
Fig. 2 is a flow chart of tests performed with the vessel of Fig. 1.

### DESCRIPTION OF PREFERRED EMBODIMENTS

Treatment (and within this specification, the term 'treatment' is intended to include within its scope, unless the specific context requires, prophylactic treatment as well as remedial treatment) of intestinal infection may vary depending upon the nature of the pathogen. In each case, however, a patient is likely to benefit from an increase in population of *Lactobacillus* spp..

According to one embodiment of the present invention, an oligosaccharide is ingested as part of a mixture which includes an edible, insoluble cellular material. In the context of this specification, the term 'insoluble' refers to the context of transmission through the alimentary canal up to the colon. Thus, if a material is insoluble in the conditions present within the alimentary canal prior to the colon for a period of time within which ordinary transmission through the alimentary canal to the colon would occur (thus, typically, though not limited to between one and ten days), then it is an insoluble material for the purposes of this specification. The edible material, in the present example, is a fibrous pulp of edible material of plant origin. The oligosaccharide is galacto-oligosaccharide (GOS). The GOS may either be infused into the pulp or simply administered in conjunction with it. The pulp may preferably, though not essentially, include fruit residue, such as may be obtained from a juicing or pressing operation. Thus the pulp may be the skins, seeds and other cellulose-containing material which remains upon the pressing or juicing of cranberries. In addition, vegetable pulp may be used, whether obtained as a residue to a juicing or pressing operation, such as may be the case with, for example, carrots, or whether generated by the mashing or liquidisation. Examples of vegetables which may serve to create a practical pulp include, but is not limited to, potatoes (for example, the skins of a baked potato), carrots, beets, celery, leeks, peppers, brassicas such as broccoli, sprouts, cauliflower and cabbage.

The infusion of oligosaccharide can be performed in a variety of ways. For example by mixing oligosaccharide GOS, in powdered or syrup form, with an edible pulp of cranberry to create a mixture in appropriate ratios. Preferably, the ratio will be in the region of 50:50 (by dry weight) fibrous pulp to oligosaccharide. The ratio can vary depending on the nature of the infection being treated, between 10:90 and 90:10 since *in vitro* experiments, described in more detail subsequently, have been found to provide differing effects depending upon the nature of the pathogen which is being treated.

Oligosaccharides, being polymers, have a greater capacity to endure in a form which can be beneficial to *lactobacillus* resident in the large intestine than simple sugars such as glucose, fructose, galactose and sucrose, these being more immediately susceptible to absorption by the host and to the bacterial digestion which takes place in the small intestine. It is hypothesised that embodiments of the invention provide for an enhanced delivery of oligosaccharides to the large intestine and, relatedly, a reduction in their assimilation further up the alimentary canal, in the stomach, duodenum or ileum. It is believed that such an enhanced delivery occurs because 'insoluble' cellular material, within which oligosaccharides are located when mixed together, while being difficult to break down higher up the alimentary canal, is easier to breakdown and more soluble to the bacteria in the colon. Breakdown of the cellular material releases the oligosaccharide which has been trapped within its cellular structure during passage through the canal thus far, and the consequent release of oligosaccharide for *lactobacillus* to thrive on. Thus, higher levels of oligosaccharide can be delivered to the colon than would be the case were the oligosaccharides introduces on their own and a corresponding increase in *lactobacillus* will result. An increase in the population of other lactic acid-producing bacteria such as, for example, *streptococcus* and *bacteriodes* may also be beneficial to the treatment of harmful intestinal infection, and such bacterial groups may also thrive a result of such a treatment.

### Salmonella

Simulated pathogenic infection of an *in vitro* pig-gut fermentation vessel with *Salmonella poona* was treated with a mixture of GOS and cranberry puree. The result was found to be that, after two days, a thousand-fold reduction in the population of *Salmonella* was obtained. It is hypothesised that, in the case of *Salmonella* a combination of at least two mechanisms provide the beneficial outcome. Firstly, the presence of GOS (which the plant material will, it is thought, *in vivo* provide for delivery to the large intestine in greater quantities than in the case where GOS is administered orally on its own) provides a favourable environment for the *lactobacillus* population, resulting in a lowering the pH and, possibly also an antibacterial effect against the *Salmonella.* Secondly, the *Salmonella* is thought to attach mechanically to the pulp. This has the effect that removal of the pulp during the course of the normal peristaltic flow within the gut will likewise remove the attached *Salmonella,* with the result that the *Salmonella* population drops. When this treatment was applied as a prophylactic *in vitro,* with subsequent infection, *Salmonella* declined to undetectable levels within three days, whereas it was retained within the population of an untreated vessel.

### Clostridium difficile

A simulated infection by *C. difficile* in an *in vitro* pig-gut fermentation vessel, when treated with a mixture of GOS and cranberry puree, resulted in a statistically significant reduction in the number of colony-forming units (cfu) of *C. difficile* within 48 hours. This is hypothesised to be attributable to an increase in numbers of *Lactobacillus,* which lower the pH and, possibly, also have an anti-bacterial effect upon the *C. difficile* than would be the case with GOS alone. The hypothesised role of the GOS being to provide a favourable environment for *lactobacilli* while the cranberry puree provides what may be thought of as a delivery mechanism to transport higher levels of GOS - and therefore undigested sugars - to the area at which an increased *lactobacillus* population would be beneficial to treatment of *C. difficile.* Although the population of *C. difficile* was not entirely eradicated, it is thought that the reduction would be of a sufficiently significant level to allow targeted methods of treatment using specific antibiotics to take effect in providing a cure, possibly in conjunction with other microflora-replacement therapies. This hypothesis is supported by anecdotal evidence, discussed subsequently.

The experiments thus far conducted would appear to indicate that, in relation to the treatment of *Salmonella* infection, the cranberry component of the mixture was the more effective. By contrast, in relation to the treatment of *C. difficile,* GOS appears to be the more active agent and has a better effect in reducing *C. difficile* population levels.

However, outside the scope of the present claimed invention, a combination of cranberry - or other edible material which includes insoluble, cellular material, such as a fibrous pulp of a different kind - and GOS produces a preparation which is likely to cover a wide range of pathogenic bacteria, since the two discussed here are from widely different taxonomic groups. This is so not least because a cranberry/GOS mixture is found to improve the ratio of *lactobacillus* to coliform bacteria to a greater extent than either substrate alone. This latter observation indicates that, for general prophylactic use a mixture of edible pulp and oligosaccharide, such as cranberry and GOS, is better than the individual components.

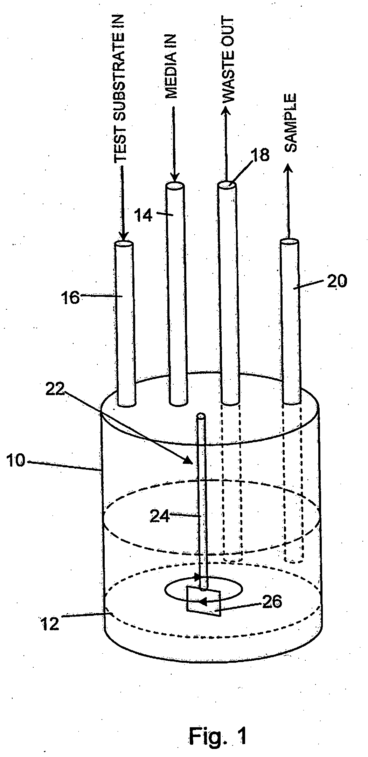

Experiments and their results, upon which the above-described hypotheses are based, will now be described. Referring now to Fig. 1, an *in vitro* simulation of pig-gut fermentation, provided by a pig-gut fermentation vessel 10 is populated, at its base, with glass beads 12, here illustrated schematically by a level within the vessel 10 below which they lie. The beads 12 simulate fronds, known as villi, within the large intestine and within which coliform and other bacteria gather - thereby avoiding being easily flushed out of the intestine during digestion and peristalsis. The vessel has two inlets 14, 16. Inlet 14 carries media which are designed to simulate the normal digestive conditions, within a large intestine. Vessels are inoculated with porcine faeces to provide the base microflora for the experiments. Thus media inlet 14 carries starches, pectins, xylan and the like. Inlet 16 carries what is known as the test substrate, i.e. the mixture or preparation whose efficacy in the treatment of pathogenic bacteria it is desired to test.

The vessel 10 also has two outlets, 18, 20. Outlet 18 provides waste removal of a combination of media and substrate, thereby to simulate the normal action of a tract of large intestine, where the contents are continually being passed through by peristalsis. The removal of this waste as fresh media is added keeps the vessel volume constant throughout the experiment. Outlet 20 is the outlet via which test samples of media are taken at various instances during the course of the experiment. Further to simulate the intestinal action, a stirrer 22, in the form of a rotating shaft 24, powered by a motor (not shown) and which rotates at a rate of approximately 60 rpm, together with a paddle 26 which lies at the distal end of the shaft 24, enhances the simulated action of the lower gut.

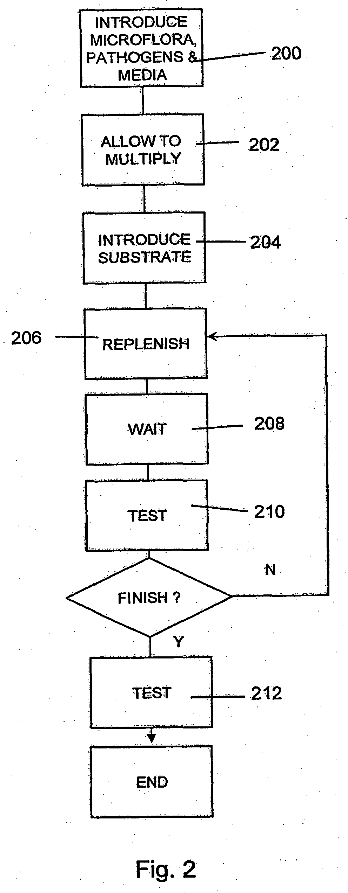

Referring now to Fig. 2, the simulation vessel was operated to provide simulation of pathogenic infection of a porcine lower intestine in the following manner.

To examine the effects of the treatment on an existing infection, the vessels are primed with a faecal inoculum to provide the microflora, fresh medium and the pathogens to be examined at step 200. Pathogens are added at this stage to allow them to be incorporated into the microflora, thereby increasing the challenge to the test substrates. The contents are then allowed to multiply and stabilise in step 202 (three days) before a further dose of pathogens is added, followed by the test substrate(s), which in the present examples comprise cranberry residue and GOS, at step 204.

Where a prophylactic effect is to be examined, the substrates are added at step 200 and daily thereafter. The pathogens are then introduced at step 204.

A detailed description of a similar system has been published in the following references, which provide more detail in relation to the operation of the system.

Hillman, K., Murdoch, T.A., Spencer, R.J. and Stewart, C.S. (1994) Inhibition of enterotoxigenic Escherichia coli by the microflora of the porcine ileum, in an in vitro semicontinuous culture system. Journal of Applied Bacteriology 76; 294-300.

Hillman, K, Spencer, R.J., Murdoch, T.A. and Stewart, C.S. (1995) The effect of mixtures of Lactobacillus spp. on the survival of enterotoxigenic Escherichia coli in in vitro continuous culture of porcine intestinal bacteria. Letters in Applied Microbiology, 20: 130-133.

Khaddour, R., Reid, C-A. and Hillman, K. (1998) Maintenance in vitro of the microflora and fermentation patterns of the porcine intestine. Pig News and Information 19: 111N-114N. Blake, D.P., Hillman, K. and Fenlon, D.R. (2003). The use of a model ileum to investigate the effects of novel and existing antimicrobials on indigenous porcine gastrointestinal microflora: using vancomycin as an example. Animal Feed Science and Technology 103: 123-139.

The initial inoculum might contain pathogens at a low level (the samples are taken from healthy individuals) but any existing pathogen will be overwhelmed by the high levels added as part of the experiment and will, in any case, be subject to the effects of the test substrates.

The flow-through effect of the gut is then simulated by replenishing the vessel contents with fresh sterile media (step 206). This is performed as three daily additions of 80% of the vessel contents at eight-hour intervals, while the waste pumps keep the total vessel volume constant. After allowing the vessel a short time to repopulate in step 208, samples are withdrawn at step 210 and the numbers of specific bacterial groups determined.

The experiment continues in this cycle until sufficient data is obtained, then one final sample is withdrawn at step 212 and the experiment terminated.

Currently, four vessels are operated simultaneously allowing for three combinations of test substrates and one control. All vessels are supplied with media from a single source to ensure there are no differences due to the feed source, and test substrates are introduced manually via port 16 (figure 1).

### EXPERIMENT 1 - Prophylactic Treatment of Salmonella with GOS and Cranberry Puree

### Materials and Methods

### Cranberry residue and GOS

The cranberry was crushed through a hand-mill prior to use, to reduce the likelihood of tubing blockage during operation of the fermentor. Cranberry was not included in the media since the substrate could not be effectively mixed, but was added daily via a separate port in the vessel lid. For consistency, the GOS and a 70:30 cranberry/GOS mixture were added to the respective vessels in the same way, all to a final concentration of 1% in the fermentor contents. Neither cranberry nor GOS were sterilised or chemically treated in any way before addition.

### In Vitro simulation.

Four fermentation vessels were employed. The working volume of each vessel was 300 ml and dilution rate was 2.4 d⁻¹ (720 ml d⁻¹). All vessels were heated in a water bath to provide 37 (±1)°C in the vessel contents. Samples were extracted through a dip tube outlet, which extended to approximately two-thirds of the depth of the fluid.

The media comprised (g l⁻¹):
xylan, 0.6; pectin, 0.6; potato starch , 5.0; casein, 3.0; peptone, 3.0; K₂HPO₄, 2.0; NaHCO₃, 1.0; NaCl, 4.5; MgSO₄.7H₂O, 0.5; CaCl₂.2H₂O, 0.45; Haemin, 0.01; Bile salts (Oxoid), 0.05; Antifoam A (0.5 ml l⁻¹) and Tween 80 (2 ml l⁻¹). All components were obtained from the Sigma group except where indicated.

The media was prepared in a 5 l reservoir containing approx 2 l of distilled water with constant (magnetic) stirring to reduce the formation of aggregates. The completed medium was made up to 5 I with distilled water, the delivery tubes were installed and the reservoir plugged with cotton-wool before sterilisation by autoclaving at 121°C for 15 minutes.

After autoclaving, the reservoirs were replaced on magnetic stirrers while hot (75-80°C); and allowed to cool with constant stirring. This prevents the formation of a carbohydrate gel in the base of the reservoir, and breaks up aggregates formed during autoclaving.

When the medium had cooled, a trace element solution (2 ml l⁻¹) and a vitamin solution (1 ml l⁻¹) were added. Stirring was provided continuously in the reservoirs throughout the experiments.

The trace element solution comprised (mg l⁻¹):
EDTA, 500; FeSO₄.7H₂O, 200; ZnSO₄.7H₂O, 10; MnCl₂.4H₂O, 3; H₃BO₃, 30; CoCl₂.6H₂O, 20; CuCl₂.2H₂O, 1; NiCl₂.6H₂O, 2; Na₂MoO₄.2H₂O, 3.

The vitamin solution comprised (mg l⁻¹):
menadione, 1; biotin, 2; sodium pantothenate, 10; nicotinamide, 5; vitamin B₁₂, 0.5; thiamine, 4; para-aminobenzoic acid, 5.

The particulate nature of the medium requires the use of wide-bore tubing, and discontinuous pump operation would result in settling of the medium within the tubes, leading to inaccurate dispensing and potential blockage. The medium was therefore pumped continuously on a recirculating loop, and diverted (by a solenoid-operated valve) to the vessels for 15 minutes every 8 hours to provide the required daily replenishment and dilution rate.

The vessels were operated for three days before the commencement of experimentation to ensure stability of the system. Test substrates were included in the vessels during the stabilisation period, except for the control (no addition), together with an inoculm of fresh porcine faeces, diluted 1:1 with pre-warmed maximum recovery diluent (Oxoid) to provide the microflora background of a healthy colon.. The arrangement of tests within vessels is shown in Table 1.

**Table 1: Fermentor operation protocol.**

| | Vessel no | | | |
|---|---|---|---|---|
| Days | 1 | 2 | 3 | 4 |
| 1-3 (no sample) | Cranberry | GOS | Cran/GOS | Control |
| 4-6 (sample set 1) | Cranberry | GOS | Cran/GOS | Control |
| 7-9 (no sample) | Control | Cranberry | GOS | Cran/GOS |
| 10-12 (sample set 2) | Control | Cranberry | GOS | Cran/GOS |
| 13-15 (no sample) | Cran/GOS | Control | Cranberry | GOS |
| 16-18 (sample set 3) | Cran/GOS | Control | Cranberry | GOS |

Vessels were all dosed with an overnight culture of *Salmonella poona* at the beginning of each sampling set.

### Bacteriological analysis.

Samples (5 ml) were taken daily for each three-day sample set. Samples were withdrawn immediately prior to the input of fresh medium so that any differences in the fermentation are maximized. After sample withdrawal and dosing with fresh media, the daily inoculum of test substrate was performed. At the start of each dataset, one sample was withdrawn to assess the starting concentration of *Salmonella poona* and the pH of the vessels immediately after the addition of fresh media.

After pH measurement, 1 ml of each sample was added to 9 ml of sterile maximum recovery diluent (MRD: Oxoid) and serially diluted to 10⁻⁶. These dilutions were then plated and incubated as shown in Table 2.

**Table 2: Media and incubation conditions for enumeration of bacterial populations.**

| Bacterial group | Media | Incubation |
|---|---|---|
| Total aerobic bacteria | Columbia brood agar. | 24 h aerobic |
| Total anaerobic bacteria | Columbia blood agar. | 48 h anaerobic |
| Coliforms | MacConkey no. 3 agar. | 24 h aerobic |
| Total lactobacilli | MRS agar | 48 h anaerobic |
| Aerotolerant lactobacilli | MRS agar | 24 h aerobic |
| Salmonella | XLD agar | 24 h aerobic |

All incubations were carried out at 37°C. Anaerobic conditions were obtained using an anaerobic jar and Oxoid Anaerogen sachets.

### Results

Samples withdrawn immediately after feeding and dosing with *Salmonella,* at the start of each run, were analysed only for pH and enumeration of *Salmonella.* These are represented in the results as 'Day 0' data.

The sample taken at the start of the second dataset was analysed in full. This was done to examine the effect of feed input on the population, and was not intended for statistical analysis. The results of this singlet is shown in Table 3.

**Table 3. A singe sample of the population taken at the start of dataset 2 (log10 cfu/ml).**

| | Total aerobes | Conforms | Aerotolerant lactobacilli | Total lactobacilli | Total anaerobes | Lac:coli |
|---|---|---|---|---|---|---|
| Cranberry | 8.78 | 8.60 | 7.16 | 7.65 | 8.74 | 0.91 |
| GOS | 8.48 | 8.30 | 8.06 | 8.32 | 8.88 | 1.00 |
| Cran/GOS | 8.65 | 8.18 | 7.65 | 8.08 | 8.65 | 0.99 |
| Control | 8.88 | 8.40 | 7.54 | 7.48 | 8.88 | 0.89 |

There appears to be little difference between the vessels immediately after feeding, so it can be assumed that the effects shown later were due to the added substrates.

Initial values of pH were determined for the growth media and for 10% suspensions/solutions of Cranberry, GOS and the 70:30 Cranberry/GOS mixture in distilled water. These are shown in Table 4.

**Table 4. Measurement of pH of the fresh growth media and of the substrates.**

| Component | Condition of test | pH |
|---|---|---|
| Fermentor media | Undiluted | 6.91 |
| Cranberry | 10% (w/v) suspension in d.H2O | 3.00 |
| GOS | 10% (w/v) solution in d.H2O | 4.05 |
| 70:30 Cranberry:GOS | 10% (w/v) suspension/solution in d.H2O | 3.34 |
| Distilled water | N/A | 6.55 |

The pH of the fermentor contents at each sample time are shown in Table 5. These are the means of triplicate data, and show an immediate drop in pH after addition of the test substrates, which is amplified over time. The control fermentation does not show a significant change in pH over the period of incubation, indicating that for normal simulation, the increased buffering is sufficient.

**Table 5. pH of samples withdrawn from the fermentation vessels.**

| Days | Cranberry | GOS | Cran/GOS | Control | SEM | P |
|---|---|---|---|---|---|---|
| 0 | 5.398^{a}ₐ | 5.05^{a}_{b} | 5.70^{a}_{c} | 6.51_{d} | 0.086 | 0.05 |
| 1 | 4.63^{b}ₐ | 3.61^{b}_{b} | 4.81^{b}ₐ | 6.21_{c} | 0.093 | 0.05 |
| 2 | 4.51^{b}ₐ | 3.47^{bc}_{b} | 4.45^{c}ₐ | 6.51_{c} | 0.175 | 0.05 |
| 3 | 4.54^{b}ₐ | 3.31^{c}_{b} | 4.38^{c}ₐ | 6.41_{c} | 0.137 | 0.05 |
| SEM | 0.143 | 0.089 | 0.134 | 0.170 | | |
| P | 0.001 | 0.05 | 0.05 | ns | | |

| | | | | | | |
|---|---|---|---|---|---|---|
| *Values within a column bearing the same superscript letter do not differ significantly (P>0.05). Values within a row bearing the same subscript letter do not differ significantly. ns - no significant differences.* | | | | | | |

Bacterial enumerations are shown in Tables 6 to 11, and the lactobacillus:coliform ratios derived from the coliform counts and the total lactobacillus counts are shown in Table 12. All bacterial counts are presented as log10 cfu/ml, and all data are the mean of triplicate experiments. The data in these tables are considered in the Discussion section.

**Table 6. Total aerobic bacteria.**

| Days | Cranberry | GOS | Cran/GOS | Control | SEM | P |
|---|---|---|---|---|---|---|
| 1 | 7.72ₐ | 7.85ₐ | 8.06_{ab} | 8.71ₐ | 0.288 | 0.05 |
| 2 | 7.90ₐ | 7.58ₐ | 8.03ₐ | 8.84_{b} | 0.331 | 0.05 |
| 3 | 7.93 | 7.68 | 7.89 | 8.62 | 0.457 ns | |
| SEM | 0.230 | 0.599 | 0.080 | 0.341 | | |
| P | ns | ns | ns | ns | | |

| | | | | | | |
|---|---|---|---|---|---|---|
| *Values within a column bearing the same superscript letter do not differ significantly (P>0.05). Values within a row bearing the same subscript letter do not differ significantly. ns - no Significant differences.* | | | | | | |

**Table 7. Total anaerobic bacteria.**

| Days | Cranberry | GOS | Cran/GOS | Control | SEM | P |
|---|---|---|---|---|---|---|
| 1 | 8.69^{a}ₐ | 8.65ₐ | 8.60ₐ | 9.14_{b} | 0.133 | 0.05 |
| 2 | 8.87^{ab}_{ab} | 8.95_{b} | 8.63ₐ | 9.24_{c} | 0.107 | 0.05 |
| 3 | 8.99^{b}_{ac} | 8.79_{ab} | 8.61_{b} | 9.22_{c} | 0.136 | 0.05 |
| SEM | 0.113 | 0.128 | 0.148 | 0.110 | | |
| P | 0.05 | ns | ns | ns | | |

| | | | | | | |
|---|---|---|---|---|---|---|
| *Values within a column bearing the same superscript letter do not differ significantly (P>0.05). Values within a row bearing the same subscript letter do not differ significantly. ns - no significant differences.* | | | | | | |

**Table 8. Total lactobacilli.**

| Days | Cranberry | GOS | Cran/GOS | Control | SEM | P |
|---|---|---|---|---|---|---|
| 1 | 8.33ₐ | 8.39ₐ | 8.20_{ab} | 7.84_{b} | 0.189 | 0.05 |
| 2 | 8.45 | 8.47 | 8.24 | 7.92 | 0.336 | ns |
| 3 | 8.54ₐ | 8.44ₐ | 8.55ₐ | 7.75_{b} | 0.214 | 0.05 |
| SEM | 0.245 | 0.155 | 0.175 | 0.380 | | |
| P | ns | ns | ns | ns | | |

| | | | | | | |
|---|---|---|---|---|---|---|
| *Values within a column bearing the same superscript letter do not differ significantly (P>0.05). Values within* a *row bearing the same subscript letter do not differ significantly. ns - no significant differences.* | | | | | | |

**Table 9. Aerotolerant lactobacilli.**

| Days | Cranberry | GOS | Cran/GOS | Control | SEM | P |
|---|---|---|---|---|---|---|
| 1 | 7.65ₐ | 8.31_{b} | 8.33_{b} | 7.59ₐ | 0.249 | 0.05 |
| 2 | 7.75_{ab} | 8.42ₐ | 8.22_{ab} | 7.67_{b} | 0.320 | 0.05 |
| 3 | 7.92 | 8.27 | 8.20 | 7.82 | 0.337 | ns |
| SEM | 0.229 | 0.075 | 0.117 | 0.547 | | |
| P | ns | ns | ns | ns | | |

| | | | | | | |
|---|---|---|---|---|---|---|
| *Values within a column bearing the same superscript letter do not differ significantly (P>0.05). Values within a row bearing the same subscript letter do not differ significantly. ns - no significant differences.* | | | | | | |

**Table 10. Coliform bacteria.**

| Days | Cranberry | GOS | Cran/GOS | Control | SEM | P |
|---|---|---|---|---|---|---|
| 1 | 7.52_{ac} | 5.96_{b} | 6.62^{a}_{ab} | 8.45_{c} | 0.417 | 0.05 |
| 2 | 6.95_{ac} | 4.80_{b} | 5.39^{b}_{ab} | 8.78_{c} | 0.805 | 0.05 |
| 3 | 7.09ₐ | 3.11_{b} | 3.97^{c}_{b} | 8.23ₐ | 1.172 | 0.05 |
| SEM | 0.837 | 1.390 | 0.482 | 0.260 | | |
| P | ns | ns | 0.05 | ns | | |

| | | | | | | |
|---|---|---|---|---|---|---|
| *Values within a column bearing the same superscript letter do not differ significantly (P>0.05). Values within a row bearing the same subscript letter do not differ significantly. ns - no significant differences.* | | | | | | |

**Table 11. Salmonella poona.**

| Days | Cranberry | GOS | Cran/GOS | Control | SEM | P |
|---|---|---|---|---|---|---|
| 0 | 7.23^{a} | 7.18^{a} | 7.26^{a} | 7.22^{a} | 0.047 | ns |
| 1 | 5.97^{b}ₐ | 4.79^{b}_{b} | 5.68^{b}ₐ | 6.93^{a}_{c} | 0.279 | 0.05 |
| 2 | ND^{c}ₐ | 3.79^{c}_{b} | 3.55^{c}_{b} | 6.77^{a}_{c} | 0.335 | 0.001 |
| 3 | ND^{c}ₐ | ND^{d}ₐ | ND^{d}ₐ | 5.95^{b}_{b} | 0.062 | 0.001 |
| SEM | 0.155 | 0.186 | 0.240 | 0.284 | | |
| P | 0.001 | 0.001 | 0.001 | 0.05 | | |

| | | | | | | |
|---|---|---|---|---|---|---|
| *Values within a column bearing the same superscript letter do not differ significantly (P>0.05). Values within a row bearing the same subscript letter do not differ significantly. ns - no significant differences.* | | | | | | |

**Table 12. Lactobacillus:coliform ratio.**

| Days | Cranberry | GOS | Cran/GOS | Control | SEM | P |
|---|---|---|---|---|---|---|
| 1 | 1.11_{ac} | 1.41_{b} | 1.25^{a}_{bc} | 0.93ₐ | 0.080 | 0.05 |
| 2 | 1.26_{ac} | 1.83_{b} | 1.55^{a}_{bc} | 0.90ₐ | 0.242 | 0.05 |
| 3 | 1.21ₐ | 1.81_{b} | 2.17^{b}_{c} | 0.94ₐ | 0.125 | 0.05 |
| SEM | 0.175 | 0.226 | 0.172 | 0.044 | | |
| P | ns | ns | 0.05 | ns | | |

| | | | | | | |
|---|---|---|---|---|---|---|
| *Values within a column bearing the same superscript letter do not differ significantly (P>0.05). Values within a row bearing the same subscript letter do not differ significantly. ns - no significant differences.* | | | | | | |

### Discussion

The ratio of total lactobacilli to coliform bacteria (Table 12) was enhanced by the addition of cranberry, although this did not reach statistical significance. Significant improvements were observed with GOS, which appeared to peak after the second day. This result is affected by a coliform value of ND (<500 cfu/ml) on day 3 of the second dataset; the apparent elimination of coliform bacteria by GOS at this point did not allow for the calculation of a lac:coli ratio. It is likely that the day-three value for GOS could be higher than is indicated here. The addition of cranberry, GOS or a combination of these to the fermentors resulted in an immediate drop in pH to around 5.0 - 5.7, all significantly lower than the control, and with the greatest immediate effect shown by the addition of GOS (Table 5). This appears unusual, since the pH of cranberry is one unit lower than that of GOS (Table 4) but probably occurs because the GOS is rapidly fermented on entering the vessel, whereas the cranberry would be used more slowly. This rapid fermentation can account for the increased acidity seen in the GOS-treated vessel.

After incubation, both GOS and the GOS/Cranberry combination showed a progressive decline in pH over three days. Even though the act of loading the vessels with fresh media appears to 'reset' the populations (Table 3), the influence of GOS and the combined substrate is cumulative. The longer these substrates are fed, the greater their effect. The maximum effect for GOS and GOS/cranberry was not reached in this experiment, although the effect of cranberry alone did not differ significantly over time, and is probably maximal at two days. It is likely that the population is adapting to the substrate, something that is likely to occur in human or animal intestines. For a human study, it might be advantageous for the subject to consume the prepared foods for a week to allow their intestinal populations to adapt before the effects are measured.

The total aerobic bacteria (Table 6) and total anaerobic bacteria (Table 7) showed little difference within vessels over time, but all treatments showed significant reductions in these total populations compared to the control. It is likely that the total would recover to untreated levels, given sufficient time, but these will be dominated by different species than in the original population. In the cranberry-treated vessel, total anaerobes have recovered over three days to the point where this population no longer differs significantly from the control (Table 7) and all populations of aerobic bacteria have reached statistical equivalence by day 3 (Table 6). These treatments are therefore likely to modify rather than reduce the total population of the colon.

The total lactobacilli were significantly increased by both cranberry and GOS after one day of fermentation (Table 8), but showed no progressive increase over time. The effects of these additives were immediate. The cranberry/GOS treatment also increased total lactobacilli, reaching significance after three days. Aerotolerant lactobacilli, a subset of the total lactobacilli, showed significant increases in population only where GOS was included (Table 9). It is likely that the primary GOS-utilising species will be within this subset, while those species encouraged by the addition of cranberry will be within the anaerobic lactobacilli. GOS and cranberry appear to enhance the growth of different species of lactobacilli.

Cranberry reduced the concentration of coliform bacteria but this did not reach significance (Table 10). Where GOS was included, the coliform bacteria were significantly reduced, and the reduction was progressive over the three sampling days. This progressive reduction was statistically significant in the cranberry/GOS treatment, suggesting that the combination was more effective than either treatment alone.

*Salmonella poona* was dosed into each vessel at the start of each three-day set, to a concentration of 10⁷ cfu/ml (Table 11). In the control, this pathogen survived at close to 10⁶ per ml after three days, but was undetectable (<500 /ml) in vessels dosed with any of the three test substrates after this time. Cranberry proved most effective at removal of *Salmonella,* with levels dropping below detectability after two days. It is possible that there is a binding action involved: the ability of cranberry to bind this pathogen has not been investigated. If this is the case, binding of the pathogen would speed its removal from the gut. All test substrates provided effective elimination of *Salmonella poona* within three days.

With the cranberry/GOS combination, the lac:coli ratio showed a progressive increase over three days, culminating in a value of 2.17, higher than that observed with either cranberry or GOS used individually. A synergistic effect is possible, although the missing datapoint in the GOS results might affect this conclusion.

It seems, then, that while both cranberry and GOS enhance lactobacilli, they enhance different groups. Therefore their use in combination is more likely to be effective than their use as individual substrates. Cranberry seems particularly effective at removing *Salmonella,* while GOS is the more effective at suppressing the general coliform population. Together, they produce a lactobacillus:coliform ratio significantly greater than either can produce alone. Both result in acidification of the intestinal fermentation which will disadvantage the coliform group while enhancing the activity of lactobacilli.

The results presented here suggest that the use of cranberry and GOS in combination is likely to be more effective in improving gut health than the use of either as single additives.

### EXPERIMENT 2 Remedial treatment of Clostridium Difficile and Salmonella Poona with GOS and Cranberry Puree

### Methods and Materials

Galacto-oligosaccharide (GOS) was included once per day in the fermentation vessels at a concentration of 1% (of product). A 50% (w/v) solution of GOS in sterile distilled water was prepared for ease of handling. The pH of this GOS solution was 3.94.

Cranberry skins (approx. 12% DM) were partially crushed using a hand-mill before use. 1% (wet weight) was added to the cranberry treatment vessel daily.

GOS/Cranberry mixture comprised a 70:30 mix of cranberry and GOS, giving 0.7% cranberry skins and 0.3% GOS as a daily addition.

### Pathogens.

The simulation was seeded with fresh *Clostridium difficile* and *Salmonella poona* cultures during its initial stabilisation phase, to allow these pathogens the best chance of establishing in the population. Further pathogen doses were added on the first day (day 0) of each run, while the treatments were not applied until the second day (day 1). The experiment was designed to make it as difficult as possible for the treatments to affect the established pathogens.

### In Vitro simulation.

The operating protocol for the current project is shown in Table 1 and includes the use of four fermentation vessels. Growth media pH averaged 6.54 over the course of the work.

**Table 1: Fermentor operation protocol.**

| | Vessel no | | | |
|---|---|---|---|---|
| Days | 1 | 2 | 3 | 4 |
| 1-3 (no sample) | Control | Cranberry | GOS | GOS/cran |
| 4-7 (sample set 1) | Control | Cranberry | GOS | GOS/cran |
| 8-10 (no sample) | Cranberry | GOS | GOS/cran | Control |
| 11-14 (sample set 2) | Cranberry | GOS | GOS/cran | Control |
| 15-17 (no sample) | GOS | GOS/cran | Control | Cranberry |
| 18-21 (sample set 3) | GOS | GOS/cran | Control | Cranberry |

### Bacteriological analysis.

Samples (5 ml) were taken daily for each three-day sample set. Samples were withdrawn immediately prior to the input of fresh medium so that any differences in the fermentation are maximized. After sample withdrawal and dosing with fresh media, the daily inoculum of test substrate was performed. At the start of each dataset, one sample was withdrawn to assess the starting concentrations of *Salmonella poona* and *Clostridium difficile* and the pH of the vessels immediately after the addition of fresh media.

After pH measurement, 1 ml of each sample was added to 9 ml of sterile maximum recovery diluent (MRD: Oxoid) and serially diluted to 10⁻⁶. These dilutions were then plated and incubated as shown in Table 2.

**Table 2: Media and incubation conditions for enumeration of bacterial populations.**

| Bacterial group | Media | Incubation |
|---|---|---|
| Total aerobic bacteria | Columbia blood agar. | 24 h aerobic |
| Total anaerobic bacteria | Columbia blood agar. | 48 h anaerobic |
| Coliforms | MacConkey no. 3 agar. | 24 h aerobic |
| Total lactobacilli | MRS agar | 48 h anaerobic |
| Aerotolerant lactobacilli | MRS agar | 24 h aerobic |
| *Salmonella* | XLD agar | 24 h aerobic |
| *Clostridium difficile* | *C. difficile* selective medium | 48 h anaerobic |

All incubations were carried out at 37°C. Anaerobic conditions were obtained using an anaerobic jar and Oxoid Anaerogen sachets. Anaerobiosis was confirmed using Oxoid indicator strips.

Where the identity of *C*. *difficile* could not be definitely ascertained by eye after incubation, colonies were tested using an antibody-based confirmation test (Oxoid).

### Results

The pH of all treated vessels declined over the course of the experiment to a greater extent than that observed in the control vessel (Table 3). GOS and GOS/cranberry treatments showed a significantly reduced pH in comparison with the control by day 2 (after one day of treatment) and by day 3, all three treatments had reduced the pH significantly in comparison with the control.

**Table 3. pH of the fermentor contents at each sample time.**

| Days | GOS | Cranberry | GOS/Cran | Control | SEM | P< |
|---|---|---|---|---|---|---|
| 0 | 6.63^{a} | 6.56^{a} | 6.68^{a} | 6.69^{a} | 0.107 | ns |
| 1 | 6.33^{a} | 6.32^{a} | 6.36^{b} | 6.51^{a}_{b} | 0.134 | ns |
| 2 | 4.73^{b}ₐ | 5.54^{b}_{bc} | 5.23^{c}_{b} | 6.14^{b}_{c} | 0.258 | 0.05 |
| 3 | 4.50^{b}ₐ | 5.17^{b}_{b} | 4.79^{d}_{ab} | 6.03^{b}_{c} | 0.158 | 0.01 |
| SEM | 0.169 | 0.189 | 0.099 | 0.216 | | |
| P< | 0.001 | 0.01 | 0.01 | 0.05 | | |

| | | | | | | |
|---|---|---|---|---|---|---|
| *Values within a column bearing the same superscript letter do not differ significantly (P>0.05). Values within a row bearing the same subscript letter do not differ significantly. ns - no significant differences.* | | | | | | |

There were no significant differences in the counts of total aerobic bacteria (table 4) or total anaerobic bacteria (Table 5) as a result of any treatment over the course of the experiment.

**Table 4. Total aerobic bacteria.**

| Days | GOS | Cranberry | GOS/Cran | Control | SEM | P< |
|---|---|---|---|---|---|---|
| 0 | 8.39 | 8.37 | 8.48 | 8.63 | 0.317 | ns |
| 1 | 8.57 | 8.74 | 8.86 | 8.76 | 0.186 | ns |
| | 8.56 | 8.68 | 8.68 | 8.82 | 0.217 | ns |
| 3 | 8.56 | 8.52 | 8.71 | 8.72 | 0.363 | ns |
| SEM | 0.252 | 0.337 | 0.288 | 0.234 | | |
| P< | ns | ns | ns | ns | | |

| | | | | | | |
|---|---|---|---|---|---|---|
| *Values within a column bearing the same superscript letter do not differ significantly (P>0.05). Values within a row bearing the same subscript letter do not differ significantly. ns - no significant differences.* | | | | | | |

**Table 5. Total anaerobic bacteria.**

| Days | GOS | Cranberry | GOS/Cran | Control | SEM | P< |
|---|---|---|---|---|---|---|
| 0 | 8.45 | 8.04 | 8.15 | 8.63 | 0.603 | ns |
| 1 | 8.38 | 8.40 | 8.35 | 8.46 | 0.388 | ns |
| 2 | 8.45 | 8.56 | 8.42 | 8.68 | 0.208 | ns |
| 3 | 8.49 | 8.20 | 8.45 | 8.56 | 0.345 | ns |
| SEM | 0.278 | 0.501 | 0.495 | 0.322 | | |
| P< | ns | ns | ns | ns | | |

| | | | | | | |
|---|---|---|---|---|---|---|
| *Values within a column bearing the same superscript letter do not differ significantly (P>0.05). Values within a row bearing the same subscript letter do not differ significantly. ns - no Significant differences.* | | | | | | |

Total lactobacilli (Table 6) were present in high numbers at the start of the experiment, Which was most likely due to a high initial inoculum in the starting sample. Their numbers declined in the control vessel over time, but there were no significant reduction over time in any vessel. GOS showed a significantly higher count of total lactobacilli by day 2; the other two treatments were higher than the control value, though the data did not reach statistical significance.

**Table 6. Total lactobacilli.**

| Days | GOS | Cranberry | GOS/Cran | Control | SEM | P< |
|---|---|---|---|---|---|---|
| 0 | 8.21 | 8.12 | 8.65 | 8.48 | 0.552 | ns |
| 1 | 8.32 | 8.43 | 8.36 | 8.37 | 0.320 | ns |
| 2 | 8.44ₐ | 8.25_{ab} | 8.02_{b} | 802_{b} | 0.162 | 0.05 |
| 3 | 8.47ₐ | 8.02_{ab} | 8.35_{ab} | 7.71_{b} | 0.340 | 0.05 |
| SEM | 0.247 | 0.410 | 0.362 | 0.435 | | |
| P< | ns | ns | ns | ns | | |

| | | | | | | |
|---|---|---|---|---|---|---|
| *Values within a column bearing the same superscript letter do not differ significantly (P>0.05). Values within a row bearing the same subscript letter do not differ significantly. ns - no significant differences.* | | | | | | |

Although the aerotolerant lactobacilli (Table 7) showed a similar pattern to the total lactobacilli (Table 6), there were few statistically significant differences, due to the wide variation in this population between runs. The initial high counts of this population also serve to mask any potential increases due to GOS or cranberry.

**Table 7. Aerotolerant lactobacilli.**

| Days | GOS | Cranberry | GOS/Cran | Control | SEM | P< |
|---|---|---|---|---|---|---|
| 0 | 7.44 | 7.65 | 7.58 | 7.31 | 1.042 | ns |
| 1 | 8.02 | 7.59 | 7.69 | 7.77 | 0.810 | ns |
| 2 | 8.22ₐ | 7.85_{b} | 8.20_{ab} | 8.07_{ab} | 0.169 | 0.05 |
| 3 | 8.30 | 8.09 | 8.37 | 7.87 | 0.380 | ns |
| SEM | 0.684 | 0.581 | 0.721 | 0.771 | | |
| P< | ns | ns | ns | ns | | |

| | | | | | | |
|---|---|---|---|---|---|---|
| *Values within a column bearing the same superscript letter do not differ significantly* (*P>0*.*05*). *Values within a row bearing the same subscript letter do not differ significantly. ns - no significant differences.* | | | | | | |

Significant declines in coliform bacteria (Table 8) were noted in all three treatments but not in the control. By day 3, the GOS and cranberry treatments had resulted in significant reduction in coliform numbers compared to the control, while the GOS/cranberry combination did not.

**Table 8. Coliform bacteria.**

| Days | GOS | Cranberry | GOS/Cran | Control | SEM | P< |
|---|---|---|---|---|---|---|
| 0 | 8.14^{a} | 8.24^{a} | 8.39^{a} | 8.12 | 0.232 | ns |
| 1 | 8.11^{a} | 8.24^{a} | 8.00^{ab} | 8.10 | 0.235 | ns |
| 2 | 8.06^{a} | 8.19^{a} | 7.78^{b} | 8.09 | 0.224 | ns |
| 3 | 7.20^{ba} | 7.18^{ba} | 7.95^{ab}_{b} | 7.85_{b} | 0.282 | 0.05 |
| SEM | 0.203 | 0.303 | 0.271 | 0.177 | | |
| P< | 0.01 | 0.01 | 0.05 | ns | | |

| | | | | | | |
|---|---|---|---|---|---|---|
| *Values within a column bearing the same superscript letter do not differ significantly (P>0.05). Values within a row bearing the same subscript letter do not differ significantly. ns - no significant differences.* | | | | | | |

*Salmonella poona* (Table 9) declined over time in all vessels. Although the decline was faster in the treated vessels than in the control, the differences did not reach statistical significance due to the high variability between runs.

**Table 9. Salmonella poona.**

| Days | GOS | Cranberry | GOS/Cran | Control | SEM | P< |
|---|---|---|---|---|---|---|
| 0 | 7.86^{a} | 8.04^{a} | 8.14^{a} | 8.19^{a} | 0.339 | ns |
| 1 | 7.05^{a} | 7.49^{a} | 7.21^{ab} | 7.24^{ab} | 0.891 | ns |
| 2 | 5.03^{b} | 5.38^{b} | 5.53^{ab} | 6.83^{ab} | 0.860 | ns |
| 3 | 4.63^{b} | 5.07^{b} | 4.90^{b} | 5.81^{b} | 1.042 | ns |
| SEM | 0.917 | 0.883 | 0.789 | 0.705 | | |
| P< | 0.05 | 0.05 | 0.05 | 0.05 | | |

| | | | | | | |
|---|---|---|---|---|---|---|
| *Values within a column bearing the same superscript letter do not differ significantly (P>0.05). Values within a row bearing the same subscript letter do not differ significantly ns - no significant differences.* | | | | | | |

*Clostridium difficile* (Table 10) showed significant reduction in numbers in all treated vessels, but not in the control. All three treatments showed significant reduction of this pathogen compared to the control by day 2, although the cranberry treatment was not significantly different from the control on day 3.

**Table 10. Clostridium difficile.**

| Days | GOS | Cranberry | GOS/Cran | Control | SEM | P< |
|---|---|---|---|---|---|---|
| 0 | | 5.70^{a} | 5.44^{a} | 5.58 | 0.356 | ns |
| 1 | | 5.00^{ab} | 5.05^{ab} | 6.10 | 1.058 | ns |
| 2 | 3.70^{b}ₐ | 4.37^{ab}ₐ | 3.70^{ab}ₐ | 5.76_{b} | 0.504 | 0.05 |
| 3 | 2.70^{b}ₐ | 3.76^{b}_{ab} | 3.37^{b}ₐ | 4.96_{b} | 0.674 | 0.05 |
| SEM | 0.489 | 0.789 | 0.849 | 0.613 | | |
| P< | 0.01 | 0.05 | 0.05 | ns | | |

| | | | | | | |
|---|---|---|---|---|---|---|
| *Values within a column bearing the same superscript letter do not differ significantly (P>0.05). Values within a row bearing the same subscript letter do not differ significantly. ns - no significant differences.* | | | | | | |

The lactobacillus: coliform ratio reached statistical significance on day 3, with all three treatments better than the control. GOS produced the highest ratio, followed by cranberry.

**Table 11. Lactobacillus:coliform ratio.**

| Days | GOS | Cranberry | GOS/Cran | Control | SEM | P< |
|---|---|---|---|---|---|---|
| 0 | 1.01^{a} | 0.98^{a} | 1.03 | 1.05 | 0.052 | ns |
| 1 | 1.03^{a} | 1.02^{a} | 1.04 | 1.03 | 0.030 | ns |
| 2 | 1.05^{a} | 1.01^{a} | 1.04 | 0.99 | 0.040 | ns |
| 3 | 1.18^{b}ₐ | 1.12^{b}_{b} | 1.05_{c} | 0.98_{d} | 0.026 | 0.05 |
| SEM | 0.033 | 0.032 | 0.043 | 0.043 | | |
| P< | 0.05 | 0.05 | ns | ns | | |

| | | | | | | |
|---|---|---|---|---|---|---|
| *Values within a column bearing the same superscript letter do not differ significantly (P>0.05). Values within a row bearing the same subscript letter do not differ significantly. ns* - *no significant differences.* | | | | | | |

### Discussion

It is clear that this experiment should have been continued for a further day on each of the triplicate runs. In future work, the period of assessment will start with the addition of treatments, which took place on day 1 of this experiment The day 0 and day 1 data show no material differences, since the day 1 samples were extracted after the vessels had been exposed to treatment for only one hour.

Nevertheless, even with only two effective days of treatment, the GOS, cranberry and combined mixture produced data which showed significant differences. All three treatments reduced *CI. difficile* numbers, all three improved the lactobacillus:coliform ratio, and all reduced the overall pH of the vessel contents.

*Salmonella poona* was reduced also although not to significant levels. Previous work has shown a prophylactic effect in that vessels pretreated with these substrates do not support the growth of *S*. *poona.* Removal of an existing infection will take longer than the two days of treatment applied here, but the trend is clear.

The high initial counts of both sets of lactobacilli were a consequence of the faecal samples used: natural samples will of course vary between pigs as they will between humans. However, even though the numbers of lactobacilli showed little variation, there was sufficient change to allow significant differences in lactobacillus:coliform ratio, and the rapid decline in pH suggests that activity of these populations was increased even though their numbers were already at, or close to, their maximum.

The experiment has shown that an existing infection of *Cl*. *difficile* can be rapidly and significantly reduced by the application of these treatments, particularly where GOS is included, and indicates that a similar effect is likely to occur with an existing infection of

*Salmonella.*

### Anecdotal Evidence Including Treatment

A patient, male of approximately 50 years old, was suffering severe symptoms of *C*. *difficile* infection, in spite of narrow-spectrum antibiotic treatment, for a period of approximately three months. The treatment protocol was then modified to include, additionally, the oral administration of cranberry puree and GOS at least once daily. The result, five days after modification of the treatment, was that no infection with *C. difficile* was possible to detect.

A further female patient, presenting for the second time with symptoms of c. *difficile* was treated initially with metronidazole and then, subsequently, with vancomycin. Neither treatment was having any effect. At the point of administration of a mixture of galacto-oligosaccharide and cranberry puree the patient was recording 16 episodes of open bowel in a 24 hour period, was suffering from severe dehydration and was causing significant concern that death might imminently result. Within 48 hours of the three-times daily oral administration of galacto-oligosaccharide and cranberry puree, the patient was recovering, with the number of open bowel episodes having reduced to three per 24 hour period.

Amongst strains of *lactobacillus,* some are more beneficially affected by oligosaccharides such as galacto-oligosaccharide than others. Given the number of different strains of *lactobacillus,* therefore, there is no reason to suppose that any given individual will necessarily have any population of those strains which are most beneficially affected. A further, independent aspect of the present disclosure, outside the scope of the invention as claimed, provides a method of generating a mixture of probiotic and prebioitic comprising the steps of treating a variety of strains of *lactobacilli* with a prebiotic oligosaccharide, identifying one or more strains of *lactobacilli* which are beneficially affected and incorporating a population of one or more of the most beneficially affected strains into a prebiotic. The resultant mixture may then be administered to persons with the aim of improving their intestinal health, thereby ensuring that they possess a population of bacteria most beneficially affected by a galacto-oligosaccharide prebiotic, for example.

During tests to examine the effect of galacto-oligosaccharide, on a population of *salmonella* (performed using the *in vitro* fermentation simulation apparatus illustrated above), it was found that the beneficial effect of the *lactobacilli* in reducing the *salmonella* population increased with increasing periods of time over which the vessel was treated with galacto-oligosaccharide. Thus, after a three week period, the action of *lactobacilli* on reducing the population of the *salmonella* was significantly increased. Subsequent tests revealed that an improvement in the efficacy of the action of the population of *lactobacilli* in reducing the *salmonella* levels continued to occur during a period in which the population of *lactobacilli* remained stable - thereby eliminating the possibility that this effect results from a simple increase in the number of *lactobacillus* present over this period of time.

This has led to a conclusion that this improved action is due to an improvement the efficacy in specific strains of *lactobacilli* population due to its treatment with the prebiotic, thereby preferentially beneficially affecting those strains which, in turn, have an enhanced action against the *Salmonella.* Three such strains of preferentially-affected *lactobacillus* which, in addition, are also effective against *salmon*e*lla* are:

### L. salivarius

### L. brevis

### L. buchneri

A further aspect of the present disclosure therefore provides for the isolation of those strains of *lactobacilli* which are thus effective by testing their efficacy against, for example, *salmonella* and *clostridium difficile,* and their recombination with galacto-oligosaccharide as a prebiotic, thereby to provide an effective food supplement having the beneficial effect of reducing the population of *salmonella* and *clostridium difficile.* Preferably, this mixture is additionally mixed with a hemi-cellular pulp. Examples of such a pulp includes cranberries or other fruit such as: blueberries, strawberries, raspberries, loganberries, gooseberries, blackcurrants, blackberries, apples, oranges, kiwi fruit, peaches, nectarines, plums, apricots, grapes and the like, as well as tomatoes, for example. Alternatively, or in addition, vegetable pulp may be used, whether obtained as a residue to a juicing or pressing operation, such as may be the case with, for example, carrots, or whether generated by the mashing or liquidisation. Examples of vegetables which may serve to create a practical pulp include, but is not limited to, potatoes (for example, the skins of a baked potato), carrots, beets, celery, leeks, peppers, brassicas such as broccoli, sprouts cauliflower and cabbage.
Thus, a further aspect of the present disclosure provides a method in which strains of most beneficially affected probiotic bacteria, such as the three indicated above, can be selected by reference to their growth, and then further selected with reference to their effect upon harmful intestinal bacteria and recombined with prebiotic.

Yet a further aspect of the present disclosure relates to the effects of infection by *c. difficile. Clostridium difficile* produces a variety of toxins. One of these, known as 'toxin A', is an enterotoxin and works directly on the gut mucosa to cause inflammation, making the cells secrete fluid. Toxin B is a cytotoxin and kills cells. Although toxin B is less important, its effects in the presence of toxin A can be harmful due to the susceptibility of the inflamed mucosal cells to its effects. Toxin A, however, is the more dangerous of the two since its effects are more apt to cause the symptoms which can, ultimately, result in septicaemia and death.

In tests on cultures of *c difficile* it was found that, over a 24 hour period, the presence of cranberry pulp at a concentration of 20% in its diluent was found to have the effect of reducing detectable toxin A levels by a factor of at least 100 times over those of the control (i.e. an identical culture without cranberry), providing very clear evidence for the beneficial effects of cranberry in reducing toxin A produced by *c. difficile.* A further aspect of the present disclosure therefore provides for the use of cranberry in producing a preparation for use in reducing toxin A levels produced by *c difficile.* A further aspect of the present disclosure provides a treatment for reducing toxin A levels produced by *c difficile* comprising the step of administering cranberry matter to a patient, orally.

The combined preparation of an edible material containing hemicellulose or other insoluble cellular components provides a preparation which has utility in treating symptoms caused by a wide range of pathogenic bacteria, since *Salmonella* and *Clostridium* are from widely different taxonomic groups. Noteworthy is that the cranberry/GOS mixture improved the lactobacillus:coliform ratio to a greater extent than either substrate alone, which would appear to indicate that, for general prophylactic use, this mixture is better than the individual components. The toxin-reducing effect of cranberry, coupled with the very significant effects in reducing the population of *c. difficile* in the *in vitro* vessels by the administration of galacto-oligosaccharide produce a combined preparation adapted to deal with the symptoms of infection by *c. difficile* by reducing the toxin levels, and also the presence of the *c*. *difficile* bacteria by boosting the levels of *lactobacillus* in the intestine. Further, there is clear evidence of the benefit of in prophylactic treatment of *salmonella* from the use of cranberry alone. However, the sole use of cranberry does not form part of the invention as claimed.

In one embodiment the cranberry and galacto-oligosaccharide can advantageously be provided via a substrate in the form of a milk-based drink such as a smoothie. Alternatively it may be provided in a substrate comprising yoghurt or a yoghurt drink. All forms of substrate may additionally include one or more of the strains of *lactobacillus* specified above.

## Claims

1. The use of a galactooligasaccharide and pulp of hemi-cellular material from cranberry in the creation of a preparation for use in treatment of infection by one of *clostridium difficile* and *salmonella,*

2. The use according to claim 1 wherein the treatment is for *salmonella* and the use further Includes the use of at least one of the following strains of bacteria in the creation of the preparation: *lactobacillus brevis, lactobacillus salivarius, lactobacillus buohneri.*

3. The use according to claim 1 or claim 2 wherein the treatment includes the treatment of enterotoxin produced by *clostridium difficile.*

4. The use according to any one of the preceding claims wherein the treatment is prophylactic treatment.

5. The use according to any one of claims 1 to 3 wherein the treatment is remedial treatment.

6. The use according to any one of the preceding claims wherein the preparation has the form of one of: packaged dried fruit or fruit bar contained in an air-tight wrapping or a drink.

## Patentansprüche

1. Die Verwendung von Galactooligosaccharid und Fruchtfleisch aus hemi-zellulärem Material von Preiselbeeren bei der Herstellung einer Zubereitung für die Verwendung bei der Behandlung einer Infektion entweder durch *Clostridium difficile* oder *Salmonella.*

2. Die Verwendung nach Anspruch 1, wobei die Behandlung für *Salmonella* ist und die Verwendung darüber hinaus die Verwendung mindestens einen der folgenden Bakterienstämme bei der Herstellung der Zubereitung umfasst: *Lactobacillus brevis, Lactobacillus salivarius, Lactobacillus buchneri.*

3. Die Verwendung nach Anspruch 1 oder nach Anspruch 2, wobei die Behandlung die Behandlung von Enterotoxin, das von *Clostridium difficile* produziert wurde, umfasst.

4. Die Verwendung nach einem der vorhergehenden Ansprüche, wobei die Behandlung eine prophylaktische Behandlung ist.

5. Die Verwendung nach einem der Ansprüche 1 bis 3, wobei die Behandlung eine Heilbehandlung ist.

6. Die Verwendung nach einem der vorhergehenden Ansprüche, wobei die Zubereitung die Form von verpacktem Dörrobst oder einer Fruchtschnitte in einer luftdichten Verpackung oder eines Getränks hat.

## Revendications

1. L'utilisation de galactooligosaccharides et de pulpe dérivés de matières hémicellulosiques de la canneberge dans la création d'une préparation à utiliser dans le traitement d'une infection à *Clostridium difficile* et à *Salmonella.*

2. L'utilisation conformément à la revendication 1 dans laquelle le traitement est indiqué pour la *salmonelle* et dont l'utilisation comprend aussi l'utilisation d'au moins l'une des souches bactériennes suivantes dans la création de la préparation : *lactobacillus brevis, lactobacillus salivarius, lactobacillus buchneri.*

3. L'utilisation conformément aux revendications 1 ou 2 dans lesquelles le traitement comprend le traitement de l'entérotoxine produite par *Clostridium difficile.*

4. L'utilisation conformément à l'une au l'autre des précédentes revendications dans lesquelles le traitement est un traitement prophylactique.

5. L'utilisation conformément à l'une ou l'autre des revendications 1 à 3 dans lesquelles le traitement est un traitement correctif.

6. L'utilisation conformément à l'une ou l'autre des précédentes revendications dans lesquelles la préparation est soit sous forme de fruits secs ou de barre fruitée emballés sous vide, soit sous forme de boisson.
